# FASCICULE DE BREVET EUROPEEN

(11) **EP 0 796 240 B1**
(45) Date de publication et mention de la délivrance du brevet: **18.04.2001**
(21) Numéro de dépôt: 95941760.1
(22) Date de dépôt: 04.12.1995
(51) Int. Cl.: C07C 235/06, C07C 233/05, C07J 9/00, A61K 47/48, A61K 48/00, C12N 15/88

(54) **LIPOPOLYAMINES COMME AGENTS DE TRANSFECTION ET LEURS APPLICATIONS PHARMACEUTIQUES**
LIPOPOLYAMINE ALS TRANSFEKTIONSMITTEL UND IHRE PHARMACEUTISCHE VERWENDUNG
LIPOPOLYAMINES AS TRANSFECTION AGENTS AND PHARMACEUTICAL USES THEREOF

(30) Priorité: 05.12.1994 FR 9414596
(43) Date de publication de la demande: 24.09.1997
(73) Titulaire: Aventis Pharma S.A., 92165 Antony Cédex (FR)
(72) Inventeur: BYK, Gerardo, F-94000 Créteil (FR); DUBERTRET, Catherine, F-92310 Sèvres (FR); SCHERMAN, Daniel, F-75645 Paris Cédex 13 (FR)
(74) Mandataire: Becker, Philippe
(86) Numéro de dépôt international: FR9501595
(87) Numéro de publication internationale: WO9617823

(56) Documents cités:
- EP-A- 0 394 111
- WO-A-94/05624
- FR-A- 2 066 157
- PROCEEDINGS OF THE NATIONAL ACADEMY OF SCIENCES OF USA, vol. 86, Septembre 1989 WASHINGTON US, pages 6982-6986, J.-P. BEHR ET AL. 'Efficient gene transfer into mammalian primary endocrine cells with lipopolyamine-coated DNA'
- BIOCONJUGATE CHEMISTRY, vol. 5, no. 6, 1994 WASHINGTON US, pages 647-654, J.-P. BEHR ET AL. 'Gene transfer with a series of lipophilic DNA-binding molecules'
- TETRAHEDRON LETTERS, vol. 27, no. 48, 1986 OXFORD GB, pages 5861-5864, J. P. BEHR 'DNA strongly bibds to micelles and vesicles containing lipopolyamines or lipointercalants'

## Description

La présente invention concerne de nouveaux composés apparentés à la famille des lipopolyamines, des compositions pharmaceutiques les contenant et leurs applications pour la transfection in vivo et/ou in vitro d'acides nucléiques.

De nombreuses maladies génétiques sont associées à un défaut d'expression et/ou une expression anormale, c'est à dire déficiente ou excessive, d'un ou plusieurs acides nucléiques. La thérapie génique a pour principal objectif de corriger ce type d'anomalies génétiques par le biais de l'expression cellulaire in vivo ou in vitro de gènes clonés.

Aujourd'hui, plusieurs méthodes sont proposées pour la délivrance intracellulaire de ce type d'information génétique. L'une d'entre elles, en particulier, repose sur l'emploi de vecteurs chimiques ou biochimiques. Les vecteurs synthétiques ont deux fonctions principales, compacter l'ADN à transfecter et promouvoir sa fixation cellulaire ainsi que son passage à travers la membrane plasmique et, le cas échéant, à travers les deux membranes nucléaires.

Un progrès important a été accompli dans ce mode de transfection avec le développement d'une technologie basée sur l'emploi d'un lipide cationique. Il a ainsi été mis en évidence qu'un lipide cationique chargé positivement, le chlorure de N-[1-(2,3-dioleyloxy)propyl]-N,N,N-triméthylammonium (DOTMA), interférait, sous la forme de liposomes ou de petites vésicules, spontanément avec de l'ADN, qui est chargé négativement, pour former des complexes lipides-ADN, capables de fusionner avec les membranes cellulaires, et permettait ainsi la délivrance intracellulaire de l'ADN. Toutefois, bien que cette molécule soit efficace au niveau de la transfection, elle présente le désavantage d'être non biodégradable et de posséder un caractère toxique à l'égard des cellules.

Depuis le DOTMA, d'autres lipides cationiques ont été développés sur ce modèle de structure : groupe lipophile associé à un groupement amino via un bras dit "spacer". Parmi ceux-ci, on peut plus particulièrement citer ceux comprenant à titre de groupement lipophile deux acides gras ou un dérivé du cholestérol, et comportant, en outre, le cas échéant, à titre de groupement amino, un groupement d'ammonium quaternaire. Les DOTAP, DOBT ou le ChOTB peuvent notamment être cités à titre représentatifs de cette catégorie de lipides cationiques. D'autres composés, comme les DOSC et ChOSC, se caractérisent par la présence d'un groupement choline à la place du groupement d'ammonium quaternaire. En général, l'activité transfectante de ces composés demeure toutefois assez faible.

Une autre catégorie de lipides cationiques, les lipopolyamines, a également été décrite dans le brevet EP 0 394 111. Dans ce type de composés, le groupement cationique est représenté par le radical L-5carboxyspermine qui contient quatre groupements d'ammonium, deux primaires et deux secondaires. Les DOGS et DPPES en font notamment partie. Ces lipopolyamines sont tout particulièrement efficaces pour la transfection de cellules endocrines primaires.

En fait, un agent de transfection synthétique idéal devrait présenter un haut niveau de transfection, et ce pour un large spectre de cellules, posséder une toxicité nulle ou à défaut une toxicité très minimisée aux doses d'utilisation, et enfin, être biodégradable pour s'affranchir de tout effet secondaire au niveau des cellules traitées.

La présente invention a précisément pour objet de proposer de nouveaux composés susceptibles d'être utilisés efficacement clans la transfection in vitro et ou in vivo de cellules et notamment pour la vectorisation d'acides nucléiques.

Elle a pour premier objet des lipopolyamines, sous forme D, L ou LD et leurs sels, représentées par la formule générale I : 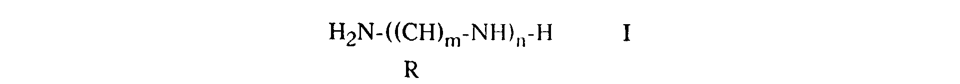 dans laquelle
- m est un nombre entier compris entre 2 et 6 inclusivement,
- n est un nombre entier compris entre 1 et 9 inclusivement et plus préférentiellement entre 1 et 5 avecun seul groupement R différent de l'hydrogène de présent dans la formule générale et des valeurs de m variables ou identiques au sein des différents groupements 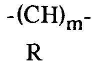 et -(CH₂)ₘ-,
- R représente un atome d'hydrogène ou un radical de formule générale II: 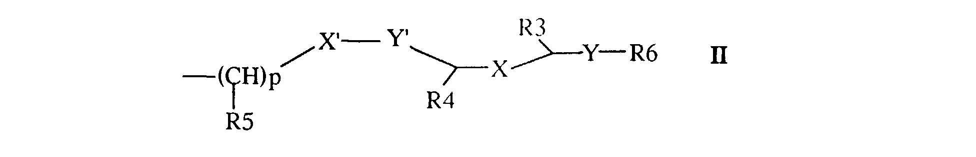
dans laquelle
- X et X' représentent, indépendamment l'un de l'autre, un atome d'oxygène, un groupement méthylène -(CH₂)_{q}- avec q égal à 0,1,2 ou 3, ou un groupement amino -NH- ou -NR'- avec R' représentant un groupement alkyle en C₁ à C₄,
- Y et Y' représentent indépendamment l'un de l'autre un groupement méthylène, un groupement carbonyle ou un groupement C=S,
- R₃, R₄ et R₅ représentent indépendamment l'un de l'autre un atome d'hydrogène ou un radical alkyle, substitué ou non, en C₁à C₄, avec p pouvant varier entre 0 et 5,
- R₆ représente un dérivé du cholestérol ou un groupement alkyle amino -NR₁R₂ avec R₁ et R₂ représentant indépendamment l'un de l'autre un radical aliphatique, saturé ou non, linéaire ou ramifié en C₁₂ à C₂₂.

D'un intérêt tout particulier sont les composés dans lesquels R y est représenté par la formule générale II' 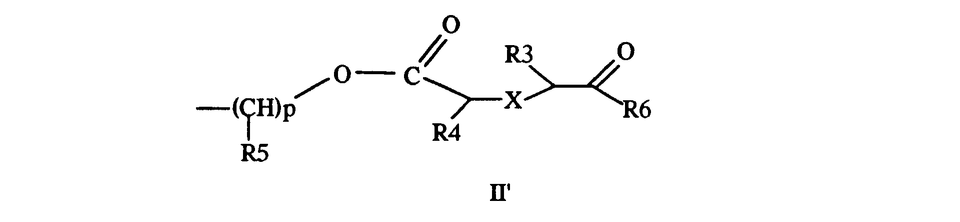 dans laquelle R₃, R₄, R₅, R₆ et p répondent aux définitions précédentes et X représente un atome d'oxygène ou un groupement -(CH₂)_{q}- avec q étant égal à zéro.

Cette famille de composés se caractérise notamment par la présence d'une liaison ester interne, intéressante sur le plan de la biodégradabilité.

A titre de lipopolyamines préférées selon l'invention, on peut plus particulièrement mentionner les composés suivants: 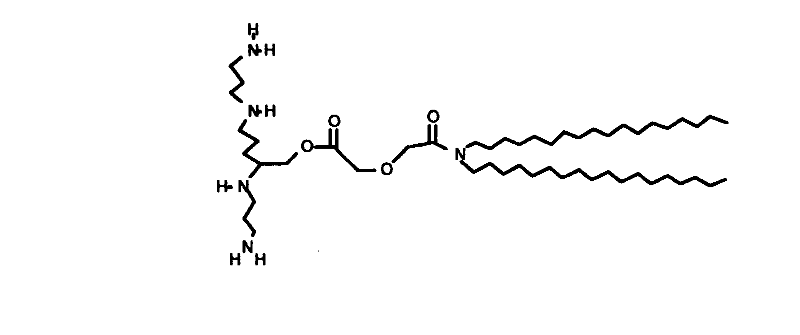 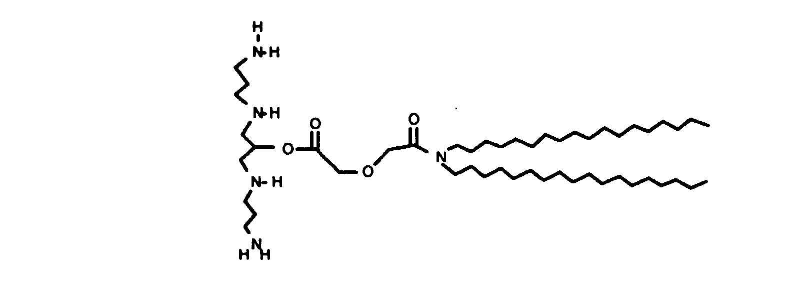 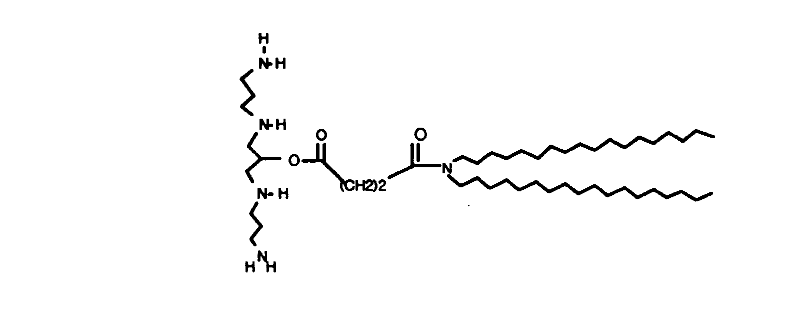 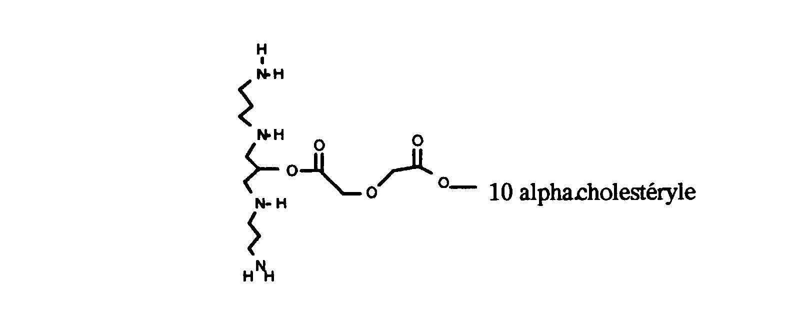 sous la forme D, L, DL ou l'un de leurs sels.

La présente invention a également pour objet toute application thérapeutique des lipopolyamines selon l'invention, soit directement, soit au sein de compositions pharmaceutiques.

Comme explicité précédemment, les composés de formule générale I s'avèrent tout particulièrement intéressants pour la transfection in vitro et in vivo d'acides nucléiques. Ils compactent efficacement l'ADN et présentent avantageusement une toxicité très réduite voire nulle à l'égard des cellules traitées. En outre, ils sont biodégradables notamment par hydrolyse de leur liaison ester.

Pour obtenir un effet maximal des compositions de l'invention, les proportions respectives du composé de formule générale I et de l'acide nucléique sont de préférence déterminées de manière à ce que le rapport R, charges positives de la lipopolyamine considérée par charges négatives dudit acide nucléique soit optimal. Ce rapport optimal variant en particulier selon le mode d'utilisation à savoir in vivo ou in vitro et selon le type cellulaire à transfecter, il est optimisé au cas par cas. Cette optimisation relève de la compétence de l'homme de l'art.

Dans les compositions de la présente invention, le polynucléotide peut être aussi bien un acide désoxyribonucléique qu'un acide ribonucléique. Il peut s'agir de séquences d'origine naturelle ou artificielle, et notamment d'ADN génomique, d'ADNc, d'ARNm, d'ARNt, d'ARNr, de séquences hybrides ou de séquences synthétiques ou semi-synthétiques. Ces acides nucléiques peuvent être par exemple d'origine humaine, animale, végétale, bactérienne ou virale. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent par ailleurs être incorporés dans des vecteurs, tels que des vecteurs plasmidiques.

Concernant plus particulièrement les acides désoxyribonucléiques, ils peuvent être simple ou double brin. Ces acides désoxyribonucléiques peuvent par exemple porter des gènes thérapeutiques, des séquences régulatrices de la transcription ou de la réplication, des séquences antisens modifiées ou non ou des régions de liaison à d'autres composants cellulaires.

Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire ayant par exemple une stabilité accrue ou une activité modifiée. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : par exemple les interleukines, les interférons ou le TNF (FR 9203120), les facteurs de croissance, les neurotransmetteurs ou leurs précurseurs ou enzymes de synthèse, les facteurs trophiques : par exemple BDNF, CNTF, NGF, IGF, GMF, aFGF, bFGF, NT3, NT5, ou HARP/pléiotrophine, la dystrophine ou une minidystrophine (FR 9111947), la protéine CFTR associée à la mucoviscidose, les gènes suppresseurs de tumeurs : par exemple p53, Rb, Rap1A, DCC ou k-rev (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidique acide phosphatase, ou encore des protéines de transfert de lipides comme la protéine de transfert des esters de cholesterol et la protéine de transfert des phospholipides, une protéine de liaisons des HDL ou encore un récepteur choisi par exemple parmi les récepteurs LDL, récepteurs des chylomicrons-remnants et les récepteurs scavenger.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus, d'autres virus ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes ElA, MLP, CMV ou RSV. En outre, ces séquences d'expression peuvent être modifiées par addition par exemple de séquences d'activation ou de régulation. Il peut aussi s'agir de promoteur, inductible ou répressible.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Dans un autre mode de mise en oeuvre, la présente invention concerne des compositions comprenant un acide nucléique, une lipopolyamine telle que revendiquée et un adjuvant capable de s'associer au complexe lipopolyamine/acide nucléique et d'en améliorer le pouvoir transfectant. La demanderesse a en effet montré que le pouvoir transfectant des lipopolyamines peut être de manière inattendue augmenté en présence de certains adjuvants (lipides ou protéines par exemple), capables de s'associer au complexe lipopolyamine/acide nucléique.

Plus préférentiellement, les compositions de l'invention comprennent, comme adjuvant, un ou plusieurs lipides neutres. De telles compositions sont particulièrement avantageuses, notamment lorsque le rapport R est faible. La demanderesse a en effet montré que l'addition d'un lipide neutre permet d'améliorer la formation des particules nucléolipidiques et, de manière surprenante, de favoriser la pénétration de la particule dans la cellule en déstabilisant sa membrane.

Plus préférentiellement, les lipides neutres utilisés dans le cadre de la présente invention sont des lipides à 2 chaînes grasses.

De manière particulièrement avantageuse, on utilise des lipides naturels ou synthétiques, zwitterioniques ou dépourvus de charge ionique dans les conditions physiologique. Il peuvent être choisis plus particulièrement parmi la dioleoylphosphatidyléthanolamine (DOPE), 1' oléoyl-palmitoylphos-phatidyléthanolamine (POPE), les di-stéaroyl, -palmitoyl, -mirystoyl phosphatidyléthanolamines ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Préférentiellement, les compositions de l'invention comprennent de 0,1 à 20 équivalents d'adjuvant pour un équivalent de composé de formule générale I et, plus préférentiellement, de 1 à 5.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire ou transdermique. De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus, soit d'un traitement de cellules en culture suivi de leur réimplantation in vivo, par injection ou greffe.

La présente invention fournit ainsi une méthode particulièrement avantageuse pour le traitement de maladies, comprenant l'administration in vivo ou in vitro d'un acide nucléique apte à corriger ladite maladie associé à un composé de formule générale I dans les conditions définies ci-avant. Plus particulièrement, cette méthode est applicable aux maladies résultant d'une déficience en un produit protéique ou nucléique et l'acide nucléique administré code pour ledit produit protéique ou contient ledit produit nucléique.

Elle s'étend à toute utilisation d'une lipopolyamine selon l'invention pour la transfection in vivo ou in vitro de cellules.

La présente invention sera plus complètement décrite à l'aide des exemples qui suivent, qui doivent être considérés comme illustratifs et non limitatifs.

### ABREVIATIONS ET SYMBOLES

- AcOEt:: Acétate d'éthyle
- BOP: Benzotriazol-1-yloxytris (diméthylamino) phosphonium hexafluorophosphate
- DCC :: Dicyclohexylcarbodiimide
- DCU :: Dicyclohexylurée
- DMAP :: 4-Diméthylaminopyridine
- DMF :: Dimélylformamide
- DMSO :: Diméthyle sulfoxide
- DODA:: Dioctadécylamine
- EP :: Ether de pétrole
- EtOH :: Ethanol
- NEt₃ :: Triéthylamine
- Rf :: Coefficient de rétention frontal
- TFA :: Acide trifluoroacétique
- THF :: Tétrahydrofurane
- TMS :: Tétraméthylsilane
- UV :: Ultra-Violets

### MATERIEL ET METHODES

### A. Produits utilisés

La DODA, la NEt₃, le TFA, la L-ornithine, l'hydroxyde de tétraméthylammonium, le nickel de Raney, l'anhydride diglycolique proviennent de chez Fluka; le BOP, de chez Propeptide France ; la DMAP, le chloroformate d'isobutyle et la N-méthylmorpholine, de chez Aidrich. Le THF provient de chez Merck ; tous les autres solvants utilisés sont des produits RP Prolabo. Les solutions de NaCl, de NaHCO₃ sont saturées ; la solution de KHSO₄ est à 0,5 M.

### B. Mesures physiques

Les spectres de résonnance magnétique nucléaire du proton ( RMN 1H ) ont été enregistrés sur un spectromètre Bruker.

Les déplacements chimiques sont exprimés en ppm par rapport au TMS.

### C. Chromatographies sur silice

. Les chromatographies sur couche mince (CCM) ont été effectuées sur des plaques de gel de silice Merck de 0,2 mm d'épaisseur.
   Révélations:
   - Aux UV (254nm)
   - A la ninhydrine, en vaporisant (spray léger) une solution éthanolique de ninhydrine ( 40 mg /100 ml EtOH) pour révéler les amines ou les amides en chauffant à 150°C.
   - A la fluorescamine, en vaporisant une solution (40 mg / 100 ml Acétone) pour révéler les amines primaires.
   - A l'iode, en recouvrant la plaque de poudre d'iode.
. Les chromatographies sur colonne ont été effectuées sur gel de silice 60 Merck de granulométrie 0,063-0,200 mm.

### EXEMPLE 1.

Synthèse du (Dioctadécyl-carbamoylméthoxy)-acétate de 2-5-bis-(3-amino-propylamino)-pentyle.

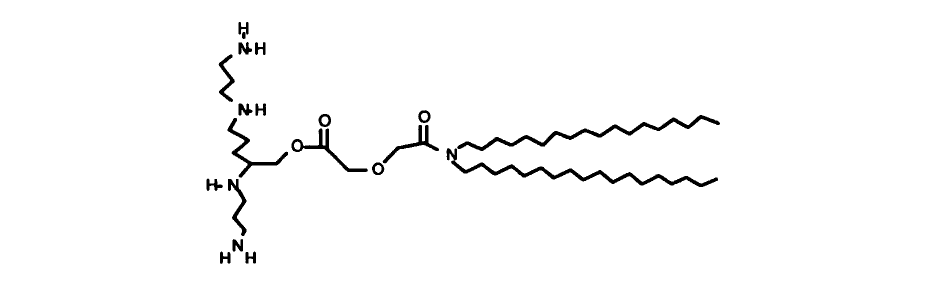

### 1.a Préparation du 2.5-Bis-(2-cyano-éthylamini)-pentanoate de tétraméthylammonium(1.a)

Du monohydrochlorure de l'acide(s)-2,5-diaminopentanoïque (L-ornithine) (6,74g; 40 mmol) et de l'hydroxyde de tétraméthylammonium pentahydraté (14,48g; 80 mmol) sont mis en solution dans du méthanol (20 ml). L'eau formée et le méthanol sont évaporés sous pression réduite (à l'aide d'une pompe à huile) de manière à obtenir un résidu sec.

Du N,N-diméthylformamide (30 ml) est ajouté aux sels précédemment obtenus. Le mélange est dégazé avec de l'azote, puis agité fortement pendant dix minutes, ce qui permet au 2,5-diamino pentanoate de tétraméthylammonium de se solubiliser (le chlorure de tétraméthylammonium reste en suspension dans le DMF).

L'acrylonitrile (6ml; 85,6 mol) est ajouté goutte à goutte; le ballon se réchauffe légèrement. Le mélange réactionnel est laissé 1h à température ambiante, sous azote. Il est ensuite filtré pour éliminer le chlorure de tétraméthylammonium. Le DMF est évaporé sous pression réduite. Le résidu obtenu est un liquide huileux ; retiré de l'évaporateur rotatif, il se solidifie. De l'acétonitrile (100 ml) est ajouté et le ballon est légèrement chauffé jusqu'à obtention d'une solution translucide. Du THF est ajouté jusqu'à obtention d'une solution trouble. Le ballon est stocké pendant deux jours au congélateur ; le sel de tétraméthyl-ammonium du L-2,5-Bis-(2-cyano-éthylamino)-pentanoïque de l'acide est récupéré par filtration sous forme de solide blanc. Il est rincé au THF sur fritté, puis séché sur P₂O₅. 6,06 g sont obtenus, soit un rendement de 49% (brut).

### 1.b Préparation du sel de tétraméthyl ammonium de l'acide 2.5-Bis-(3-amino-propylamino)-pentanoïque (1.b)

Le produit 1.a est solubilisé dans un mélange d'éthanol (18 ml), d'eau (2 ml) et de potasse 2 M (5 ml). La solution est purgée à l'argon. Du Nickel de Raney (2 ml de la suspension Fluka) est ajouté. L'hydrogénation est réalisée dans une autoclave, purgée à l'azote, à 26°C. En 3h, la pression est passée de 50,6 bars à 44,7 bars, puis s'est stabilisée plus d'une heure. L'autoclave fait 250 ml et le mélange réactionnel, 30 ml. La suspension obtenue est filtrée, rincée à l'eau et passée à l'évaporateur rotatif. Une huile jaune est obtenue. Le test à la fluorescamine est positif.

### 1.c Préparation de l'acide 2.5-Bis-{tert-butoxycarbonyl-[3-(tert-butoxycarbonyl-amino)-propyl]-amino}-pentanoïque (1.c)

Le produit 1.b est dissous dans du dioxanne (30 ml). Du ditertbutyldicarbonate (17,46g ; 80 mmol) est ajouté goutte à goutte. Le mélange est agité pendant une nuit. Le dioxanne est évaporé. Du KHSO₄ est ajouté. Le produit est extrait par CHCl₃ (3 x 100 ml). La phase organique est ensuite lavée successivement avec NaHCO₃ (pH=7,5), NaCl, puis séchée sur MgSO₄ et évaporée sous pression réduite. 10 g de produit sont obtenus (15,4 mmol, soit un rendement de 39% par rapport à l'ornithine).
**CLHP:** MeCN/H₂O: 0-3 min 50 % MeCN; 3-20 min 50-100 % MeCN, 20-40 min 100 % MeCN, K'= 7.96 (Colonne RP-18, debit=1 ml/min)
**CCM:** Rf (CHCl₃: AcOEt ; 95 : 5 (v : v)) =0,28
**RMN**(ppm): 1,3 (m, 8H, N+CH₂-**CH**_{**2**}-**CH**_{**2**}-CHN+COO/ 2x N+CH₂**CH**_{**2**}CH₂N+); 2,7-3.0 (m, 10 H 5x N+**CH**_{**2**}); 3,8 (t, 1H, N+C**H**COO)
**MS:**MH⁺= 647, (PM= 646)

### 1.d Préparation du 2.5-Bis-{tert-butoxycarbonyl-[3-(tert-butoxycarbonylamino)-propyl]-amino}-pentan-1-ol (1.d)

La L-5-carboxytétratert-butoxycarbonylspermine (1,94g; 3 mmol) est dissoute dans 30 ml de THF. 370 ml (3,1 mmol) de N-méthylmorpholine sont introduits à la micropipette. Le mélange réactionnel, mis sous azote, est refroidi à -15°C (dans un bain de carboglace et d'éthylène glycol) ; 390 ml (3,1 mmol) de chloroformate d'isobutyle sont alors ajoutés. Au bout de trois minutes, le mélange réactionnel est versé dans un becher contenant le NaBH₄ (2g) dissous dans 20 ml d'eau à 4°C. Le THF est évaporé. Du KHSO₄ est ajouté (jusqu'à Ph=7). Le produit est extrait à l'AcOEt, rincé au NaHCO₃, NaCl, séché sur MgSO₄, filtré puis évaporé. 1,15g sont obtenus, soit un rendement de 61%.

La CCM conduit à deux tâches ; une séparation sur colonne de silice, avec le même éluant, est donc réalisée et conduit à 0,97g de produit (huile jaune). Le rendement de la séparation est de 84 %.

Le rendement de la réduction est de 51 %.
**CCM:**Rf(EP : AcOEt ; 1 : 1 (v:v)) =0,24
**RMN** (ppm) : 1,42 (s, 4xBoc); 1,5-1,7 (m, 8H N+CH₂-**CH**_{**2**}-**CH**_{**2**}-CHN/ 2x N+CH₂**CH**_{**2**}CH₂N); 2,85-3,2 (m, 10H 5x **NCH**_{**2**}) , 3,4 (m, 1H, N**CH**CH₂OH), 3,7 (d, 2H, CH₂OH), 6.8 (m, 2H; **NH**)
**MS** : MH⁺= 633, PM= 632

### 1.e Préparation de l'acide 2.5-Bis-[3-(tert-butoxycarbonylamino)-propyl]-{tert-butoxycarbonylamino}-pentyloxycarbonylméthoxy-acétique (1.e)

0,51 g (0,806 mmol) du produit 1.d sont dissous dans 10 ml de CH₂Cl₂; 2 équivalents d'anhydride diglycolique (1,535 mmol; 0,178 g), 2,2 équivalents de triéthylamine (1,687 mmol; 235 µl) ainsi que 5 mg de DMAP sont ajoutés. Au bout d'une heure, la C.C.M. montrant que l'alcool a réagi, le mélange est additionné de CH₂Cl₂, puis lavé par 3 x 50 ml de KHSO₄, 3 x 50 ml de NaCl, séché sur MgSO₄, filtré puis évaporé. 0,26 g de solide sont obtenus, soit un rendement de 43 %.
**CCM** : Rf (CHCl₃ : MeOH : AcOH ; 90 : 8 : 2 (v : v : v )) =0,75
**MS**: MH+= 749, PM= 748

### 1.f. Préparation du Dioctadecyl-carbamoylmethoxy)-acétate du 2-5-bis-{tert-butoxycarbonyl-[3-(tert-butoxycarbonyl-amino)-propyl]-amino}-pentyle (1.f)

0,123 g de produit précédent (0,164 mmol), 1 équivalent (0,0856g) de dioctadécylamine, 3 équivalents de triéthylamine (68,4 ml), 1,1 équivalent (0,0798 g) de BOP sont dissous dans du CHCl₃. Le mélange réactionnel est agité à température ambiante. Au bout de deux heures, le mélange est additionné de 100 ml de CH₂Cl₂, puis lavé par 3 x 100 ml de KHSO₄, de NaHCO₃, de NaCl (jusqu'à pH=7), séché, puis évaporé. 0,13 g de produit sont obtenus, soit un rendement de 63 %.
**CCM** : Rf (EP : AcOEt; 1 : 1 (v : v)) =0,62 ; révélation à l'iode, à la ninhydrine et à la fluorescamine.
**RMN** (ppm): 0.90 (t, J=7.5 Hz, 6H: CH₃, 1.20-1.75 (m, 108 H: CH₂ / C(CH₃)₃), 3.05-3.35 (m, 14 H: CH₂N), 4.15-4.35 (m, 3 H: NCHCH₂OCO), 4.23-4.27 (2s, 2x
2H: OCH₂CO), 4.5-5.5 (m, étalé, 2 H: NH)
**MS**: MH⁺= 1252, P.M.= 1251
**Analyse élémentaire :**
Formule brute : C₇₁H₁₃₇N₅O₁₂
% théoriques: C 68,06 H 11,02 N 5,59
% obtenus: C 67,14 H 11,93 N 5,86

### 1.g. Préparation du (Dioctadecyl-carbamoylmethoxy)-acétate de 2-5-bis-(3-amino-propylamino)-pentyle (1.g)

1 ml de TFA est ajouté à 0,025 g de produit 1.f (0,02 mmol) dans un tube "Eppendorf" ® de 1,5 ml et laissé une heure à température ambiante. Le TFA est évaporé.

500 µl d'éthanol sont ajoutés de façon à obtenir une solution à 40 mM, nécessaire pour les tests biologiques.
**MS** : MH+=852, P.M.= 851

### EXEMPLE 2.

Préparation du (Dioctadécyl-carbamoylméthoxy)-acétate de 1,3-bis-(3-amino-propylamino)-2 propyle (2.f.):

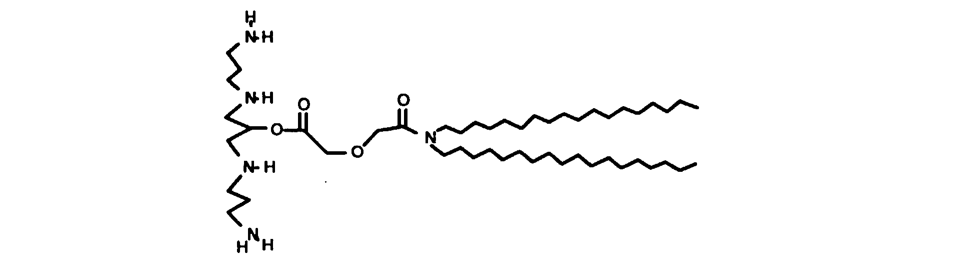

### 2.a Préparation du 1.3-Bis-(2-cyano-éthylamino)-propan-2-ol (2.a)

3,6 g (40 mmol) de 1,3-diaminopropan-2-ol sont dissous dans 75 ml de méthanol. 5,76 ml (80 mmol) d'acrylonitrile sont ajoutés en 15 minutes. Le test à la fluorescamine est positif. Le mélange réactionnel reste incolore. Il est laissé sous agitation, à température ambiante, pendant la nuit. Le test à la fluorescamine est alors négatif. Le méthanol est évaporé. 7,9 g de produit sont recueillis (rendement de 100 % pour le produit brut).

### 2.b Préparation du 1.3-Bis-(3-amino-propylamino)-propan-2-ol (2.b)

L'hydrogénation est réalisée dans une autoclave, purgée à l'azote, à 27°C.

Le produit 2.a est solubilisé dans un mélange de 15 ml de méthanol, 10 ml d'éthanol, 5 ml de KOH (2 M). La solution est purgée à l'argon et 4 ml de la suspension de nickel de Raney sont ajoutés dans l'autoclave.

En 3h30, la pression est passée de 51,6 bar à 37,6 bar, puis s'est stabilisée plus d'une heure. L'autoclave fait 250 ml et le mélange réactionnel, 30 ml. La suspension obtenue est filtrée, rincée à l'eau et passée à l'évaporateur rotatif. Une huile jaune est obtenue. Le test à la fluorescamine est positif.

### 2.c Préparation du 1.3-Bis-{3-tert-butoxycarbonyl-amino-(tert-butoxycarbonyl-aminopropyl}2-propan-2-ol (2.c)

Le produit 2.b est protégé de la même façon qu'en 1.c. 100 ml de CH₂Cl₂ sont ajoutés. 39,2 g (179 mmol) de di-tert-butyldicarbonate dissous dans 100 ml de dioxanne sont ajoutés goutte à goutte. La solution est laissée sous agitation pendant 72h. Le solvant est évaporé, du KHSO₄ est ajouté. Le produit est extrait à l'AcOEt (3 x 100 ml); les phases sont rassemblées et rincées au KHSO₄, au NaHCO₃, au NaCl, séchées sur MgSO₄ et évaporées. 20 g sont obtenus (rendement de 83 % par rapport au produit initial)
Le produit est cristallisé dans l'EP.
**C.C.M. :**
Rf (EP : AcOEt; 1 : 1 (v : v)) = 0,23
Rf (EP : AcOEt ; 1 : 2 (v : v)) = 0,63
**RMN** (ppm) : 1,4 (2s, 36H : 4 x (CH₃)₃)) ; 1,65 (qt, 4H : 2 x NCH₂C**H**_{**2**}CH₂NH); 2,95 (q, 4H : 2 x NHC**H**_{**2**}CH₂) ; 3,1 (2d, 4H : NC**H**_{**2**}CHC**H**_{**2**}N) ; 3,2 (t, 4H:
NC**H**_{**2**}CH₂) ; 3,85 (m, 1H : 0H) ; 5,9 (s, 2H : NH)
**MS**: MH⁺=605, PM= 604

### 2.d Préparation de l'acide 1,3-Bis-{3-tert-butoxycarbonyl-amino-(tert -butoxycarbonyl-aminopropyl}2-propyl-2-éthoxycarbonylméthoxy-acétique (2.d.)

604 mg (1mmol) de produit 2.c. sont dissous dans 20 ml de CH₂Cl₂. 2 équivalents d'anhydride (2 mmol ; 0,232 g), 2,2 équivalents de NEt₃ ( 307 µl, puis 100 µl), 6,5 g de DMAP sont introduits. Après 24 h d'agitation à température ambiante, le mélange est additionné de CH₂Cl₂, lavé au KHSO₄, au NaCl, séché sur MgSO₄, filtré puis évaporé. 0,156 g de produit sont obtenus (rendement de 22 % ).
**CCM** : Rf (CHCl₃ : MeOH : AcOH ; 90 : 8 : 2 {v : v : v}) =0,71
**RMN** (ppm): 1,2-1,4 (2s, 36H : 4 X (CH₃)₃) ; 1,5 (m, 4H : 2 x NHCH₂C**H**_{**2**}CH₂N) ; 2,85 (q, 4H : 2 x NHC**H**_{**2**}CH₂) ; 3,1 (m, 8H : 4 x NCH₂) ; 3,9-4,2 (2s, 4H : 2 x
OCH₂COO) ; 5,25 (m, 1H: CH₂C**H**CH₂) ; 6,7 (2H : NH)
**MS** : MH+= 721, PM= 720

### 2.e Préparation du (Dioctadécyl-carbamoylméthoxy)- acétate du 1.3-Bis-{3-tert -butoxycarbonyl-amino-(tert-butoxycarbonyl-aminopropyl} 2-propyle (2.e)

0,216 mmoles de l'acide 2.d sont dissoutes dans 3 ml de CHCl₃. 1 équivalent de DODA (0,113 g), 3 équivalents de NEt₃ (100 µl) et 1,1 équivalent de BOP (0,11 g) sont ajoutés. Le mélange réactionnel est agité à température ambiante pendant 24 h. Le CHCl₃ est évaporé. 100 ml d'AcOEt sont ajoutés. La phase organique est lavée par de l'HCl (0,5 M), par NaHCO₃, par NaCl jusqu'à pH=7, séchée sur MgSO₄ puis évaporée. 0,185 g sont obtenus (rendement 70 % ). Une colonne sur silice est réalisée. 110 mg de produit sont recueillis (rendement 42%).
**CCM** :
Rf (CHCl₃ : MeOH : AcOH; 90 : 8 : 2 (v : v : v)) =0,81
Rf (EP : AcOEt ; 1 : 1 (v : v )) = 0,67
**RMN** (ppm) : 0,85(t, 6H : 2 x CH₃) ; 1,20-1,55 (m, 100H : CH₂/C(CH₃)₃) ; 1,6 (m, 4H : 2 x NHC**H**_{**2**}CH₂CH₂N) ; 3,05-3,4 (m, 16H : 2 x NHC**H**_{**2**}CH₂C**H**_{**2**}N / NC**H**_{**2**}CHC**H**_{**2**}N / 2 x CH₂N) ; 4,1 (s, 2H : NCOCH₂O) ; 4,15 (s, 2H : OCH₂COO) ; 5,25 (m, 1H : CH₂**CH**CH₂), 6,15 (m, 2H : NH)
**MS** : MH+= 1224, PM= 1223

### 2.f Préparation du (Dioctadécyl-carbamoylmethoxy)-acétate de 1.3-Bis-{3-tert-butoxycarbonyl-amino-(tert-butoxycarbonyl-aminopropyl} 2-propyle(2.f.):

1 ml de TFA est ajouté à 0,0247 g de produit 2.e (0,021mmol) dans un tube "Eppendorf" ® de 1,5 ml et laissé une heure à température ambiante. Le TFA est évaporé.

505 µl d'éthanol sont ajoutés de façon à obtenir une solution à 40 mM, nécessaire pour les tests biologiques.
**MS** : MH⁺= 824, P.M.=

### EXEMPLE 3 : Préparation du (N,N-Dioctadécyl)-succinamate de 2-(3-amino-propylamino)-1-(3-amino-propylamino-methyl)-éthyle

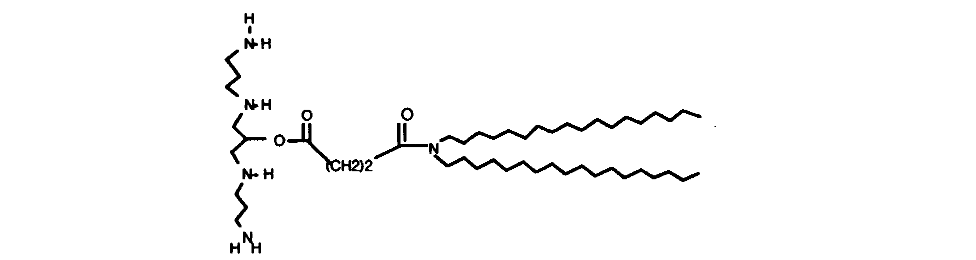

Ce composé est préparé selon le protocole décrit précédemment en exemple 2, en substituant à l'acide diglicolique, l'acide succinique.

L'analyse en spectrométrie de masse du produit ainsi obtenu indique un fragment MH⁺ de 808, ce qui est conforme à la masse attendue.

### UTILISATION DE LIPOPOLYAMINES SELON L'INVENTION POUR LA TRANSFECTION IN VITRO DE MATERIEL GENETIQUE

### A Plasmides utilisés pour le transfert de gènes in vitro

On utilise le plasmide pCMV-LUC. Il s'agit d'une construction comportant le gène rapporteur "luciférase", dérivée soit du plasmide pGL2-Basic Vector^{®} (Promega) soit du plasmide pGL2-Control Vector^{®} (Promega) par insertion d'un fragment Mlu I-Hind III contenant le promoteur du Cytomeagalovirus humain (CMV), extrait du plasmide vecteur pcDNA3^{®} (Invitrogen).

### B Protocole de préparation des solutions utilisées pour la transfection

Des lipopolyamines préparées selon des exemples précédents sont mises en solution à 40 mM dans l'éthanol puis dilués dans un mélange éthanol/eau en maintenant une concentration éthanolique inférieure à 10%.
Les solutions d'acide nucléique à transfecter sont diluées en sérum physiologique (NaCl 0,15M) puis ajoutées aux solutions de lipopolyamine, dans un rapport 1/1 (v/v). Après homogénéisation au vortex et incubation 15 minutes à température ambiante, les solutions ADN/lipopolyamine sont distribuées à 9% (v/v) final dans les puits où les cellules ont été lavées par du milieu de culture dépourvu de protéines (sérum).

### EXEMPLE 4 :

### Influence du rapport de charge (amines/phosphates) sur l'efficacité de transfection.

Des échantillons de 1.10⁵ cellules NIH 3T3 en phase exponentielle de croissance sur 2 cm² sont traités par des solutions lipopolyamines/pCMV-LUC, présentant des rapports de charges variables, pendant 4 heures à 37°C sous 5% CO₂ ; chaque échantillon reçoit 1 µg d'acide nucléique. La recherche de l'expression du gène reporter est effectuée après addition de sérum de veau foetal à 8% final suivie d'une incubation de 40 heures dans l'étuve à CO₂.
L'activité luciférase est dosée par l'émission de lumière [RLU = relative light unit] en présence de luciférine, coenzyme A et ATP pendant 20 secondes et rapportée au µg de protéine dans le surnageant obtenu après lyse des cellules. Les résultats obtenus sont reportés dans le tableau I ci-dessous.

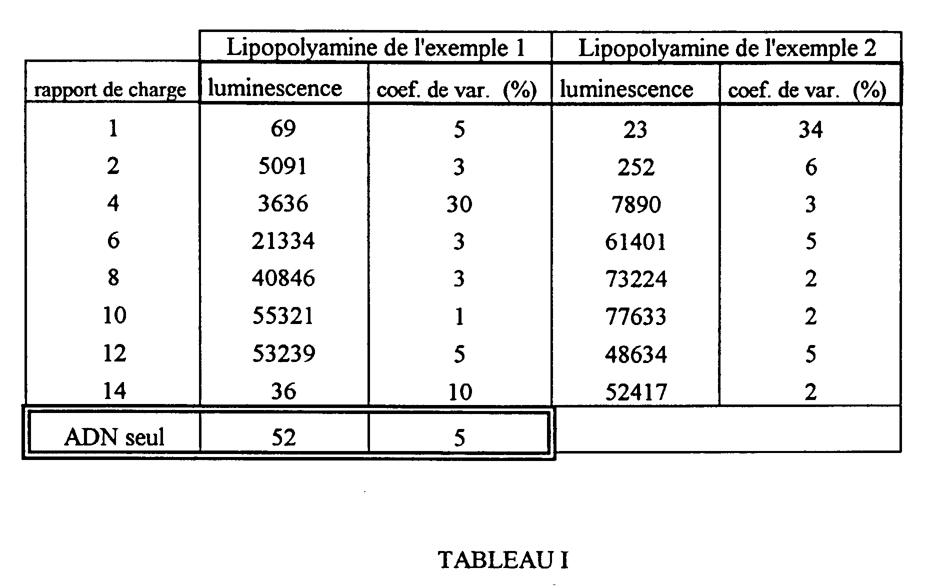

Chaque valeur correspond à la moyenne de trois essais indépendants.

Cette expérience montre clairement que les lipopolyamines selon l'invention permettent le transfert de gènes dans les conditions requises pour leur expression.

### EXEMPLE 5 :

### Influence de la concentration en acide nucléique dans les mélanges ADN/lipopolyamine.

Selon le même protocole que celui décrit dans l'exemple précédent les cellules NIH 3T3 sont traitées avec des mélanges ADN/lipopolyamines dans les conditions où différentes concentrations d'ADN sont utilisées pour un même rapport de charge.

Dans ce cas, l'activité luciférase est mesurée pendant 20 secondes et rapportée à 2,5.10³ cellules traitées. Les résultats sont présentés en tableaux II et III ci-dessous.

Lipopolyamine de l'exemple 1

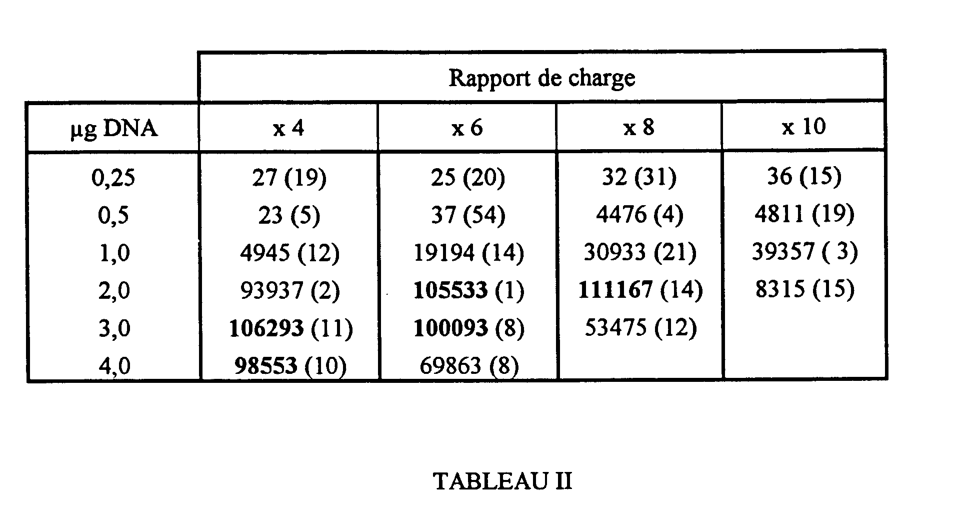

Lipopolyamine de l'exemple 2

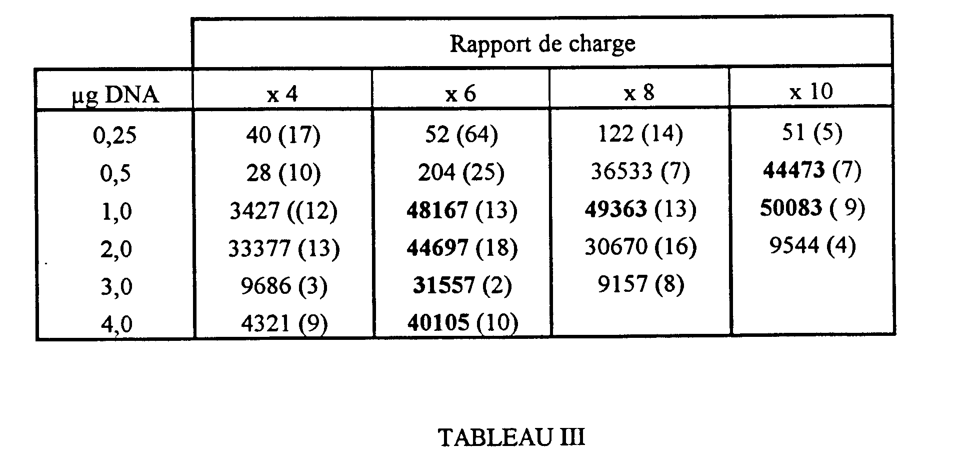

Chaque valeur correspond à la moyenne de trois essais indépendants.

Les valeurs portées entre parenthèses correspondent au coefficient de variation exprimé en %.

## Revendications

1. Lipopolyamine sous forme D, L, ou DL ou l'un de ses sels caractérisée en ce qu'elle est représentée par la formule générale I 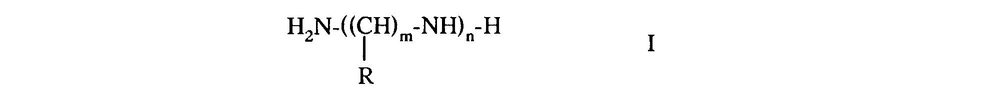 dans laquelle
- m est un nombre entier compris entre 2 et 6 inclusivement,
- n est un nombre entier compris entre 1 et 9 inclusivement avec nécessairement un seul groupement R différent de l'hydrogène de présent dans la formule générale et des valeurs de m variables ou identiques au sein des différents groupements-(CH₂)ₘ- ou 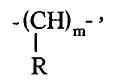
- R représente un atome d'hydrogène ou un radical de formule générale II: 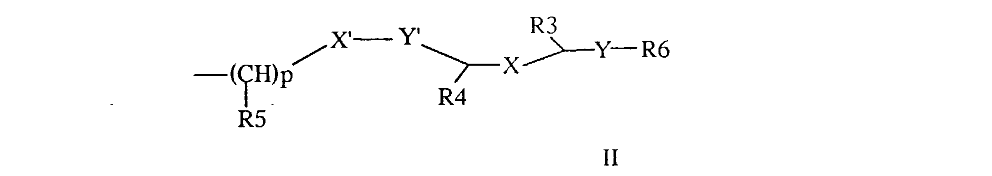 dans laquelle
- X et X' représentent indépendamment l'un de l'autre un atome d'oxygène, un groupement méthylène -(CH₂)_{q}- avec q égal à 0, 1, 2 ou 3, ou un groupement amino -NH- ou -NR'- avec R' représentant un groupement alkyle en C₁ à C₄,
- Y et Y'représentent indépendamment l'un de l'autre un groupement méthylène, un groupement carbonyle ou un groupement C=S,
- R₃, R₄ et R₅ représentent indépendamment l'un de l'autre un atome d'hydrogène ou un radical alkyle, substitué ou non, en C₁à C₄, avec p pouvant varier entre 0 et 5,
- R₆ représente un groupement cholestérol ou un groupement alkyle amino -NR₁R₂ avec R₁ et R₂ représentant indépendamment l'un de l'autre un radical aliphatique, saturé ou non, linéaire ou ramifié en C₁₂ à C₂₂.

2. Lipopolyamine selon la revendication 1 caractérisée en ce que n est choisi plus préférentiellement entre 1 et 5.

3. Lipopolyamine selon la revendication 1 caractérisée en ce que R y est représenté de préférence par la formule générale II' 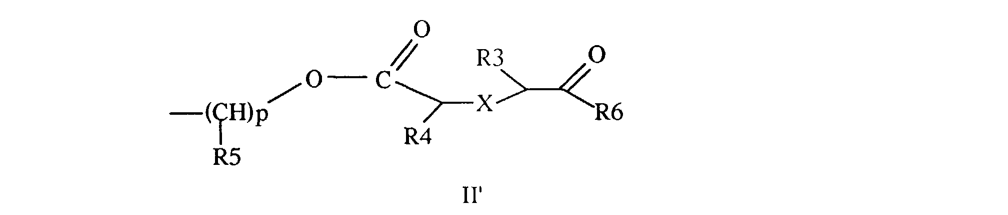 dans laquelle R₃, R₄, R₅, R₆ et p répondent aux définitions proposées en revendication 1 et X représente un atome d'oxygène ou un groupement -(CH₂)_{q}- avec q étant égal à zéro.

4. Lipopolyamine selon la revendication 1 ou 3 caractérisée en ce qu'il s'agit du 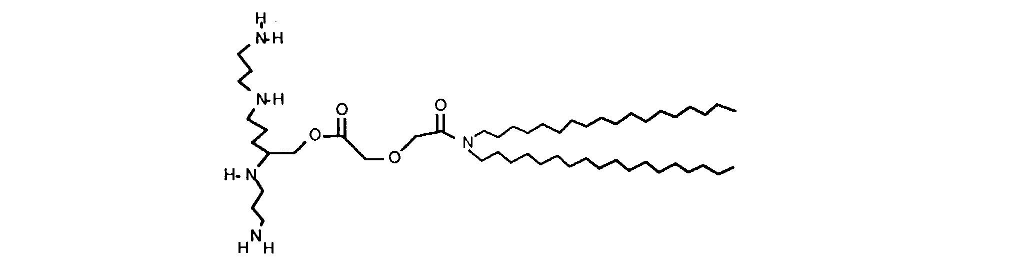 sous forme D, L, DL ou l'un de ses sels.

5. Lipopolyamine selon la revendication 1 ou 3 caractérisée en ce qu'il s'agit du 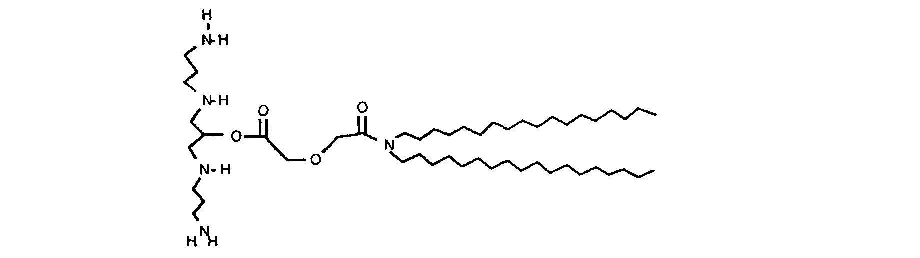 sous forme D, L, DL ou l'un de ses sels.

6. Lipopolyamine selon la revendication 1 ou 3 caractérisée en ce qu'il s'agit du 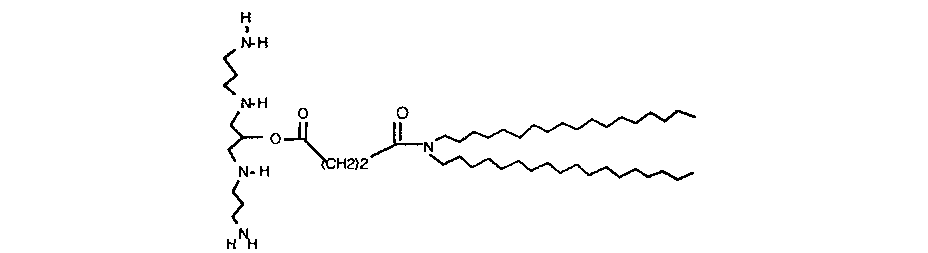 sous forme D, L, DL ou l'un de ses sels.

7. Lipopolyamine selon la revendication 1 ou 3 caractérisée en ce qu'il s'agit du 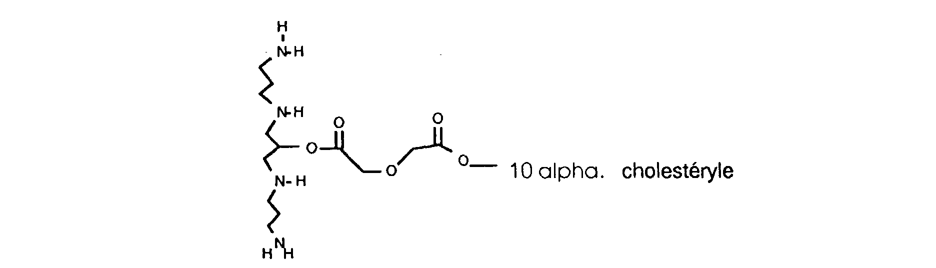 sous forme D, L, DL ou l'un de ses sels.

8. Composition pharmaceutique caractérisée en ce qu'elle contient au moins une lipopolyamine selon l'une des revendications 1 à 7 et au moins un acide nucléique.

9. Composition selon la revendication 8 caractérisée en ce que l'acide nucléique est un acide désoxyribonucléique.

10. Composition selon la revendication 8 caractérisée en ce que l'acide nucléique est un acide ribonucléique.

11. Composition selon la revendication 8, 9 ou 10 caractérisée en ce que l'acide nucléique est modifié chimiquement.

12. Composition selon l'une des revendications 8 à 11 caractérisée en ce que l'acide nucléique est un antisens.

13. Composition selon l'une des revendications 8 à 11 caractérisée en ce que l'acide nucléique comporte un gène thérapeutique.

14. Composition selon la revendication 13 caractérisée en ce que le gène thérapeutique code pour une protéine impliquée dans le métabolisme des lipides comme par une apolipoprotéine choisie parmi les apolipoprotéines A-I, A-II, A-IV, B, C-I, C-II, C-III, D, E, F, G, H, J et apo(a), une enzyme telle la lipoprotéine lipase, la lipase hépatique ou autres lipases, la lécithine cholestérol acyltransférase, la 7 alpha cholestérol hydroxylase, la phosphatidique acide phosphatase, une protéine de transfert de lipides comme la protéine de transfert des esters de cholestérol et la protéine de transfert des phospholipides, une protéine de liaisons des HDL ou encore un récepteur choisi par exemple parmi les récepteurs LDL, récepteurs des chylomicrons-remnants et les récepteurs scavenger.

15. Composition pharmaceutique comprenant un acide nucléique, une lipopolyamine selon l'une des revendications 1 à 7 et un adjuvant capable de s'associer au complexe lipopolyamine/acide nucléique et d'améliorer son pouvoir transfectant.

16. Composition selon la revendication 15 caractérisée en ce que l'adjuvant est un ou plusieurs lipides neutres.

17. Composition selon la revendication 16 caractérisée en ce que le ou les lipides neutres sont choisis parmi les lipides synthétiques ou naturels, zwitterioniques ou dépourvus de charge ionique dans les conditions physiologiques.

18. Composition selon la revendication 17 caractérisée en ce que le ou les lipides neutres sont des lipides à 2 chaînes grasses.

19. Composition selon la revendication 18 caractérisée en ce que le ou les lipides neutres sont choisis parmi la dioléoylphosphatidyléthanolamine (DOPE), l'oléoyl-palmitoylphos-phatidyléthanolamine (POPE), le di-stéaroyl, -palmitoyl, -mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) et les asialogangliosides (tels que notamment les asialoGM1 et GM2).

20. Composition selon l'une des revendications 15 à 19 caractérisée en ce qu'elle comprend de 0,1 à 20 équivalents d'adjuvant pour 1 équivalent de lipopolyamine, et, plus préférentiellement, de 1 à 5.

21. Composition selon l'une quelconque des revendications 8 à 20 caractérisée en ce qu'elle comprend un véhicule pharmaceutiquement acceptable pour une formulation injectable.

22. Composition selon l'une quelconque des revendications 8 à 20 caractérisée en ce qu'elle comprend un véhicule pharmaceutiquement acceptable pour une application sur la peau et/ou les muqueuses.

23. Utilisation d'une lipopolyamine selon l'une des revendications de 1 à 7 pour préparer une composition pour la transfection in vivo ou in vitro de cellules.

## Patentansprüche

1. Lipopolyamin in der Form D, L oder DL oder eines seiner Salze, dadurch gekennzeichnet, daß es durch die folgende allgemeine Formel (I) dargestellt wird: 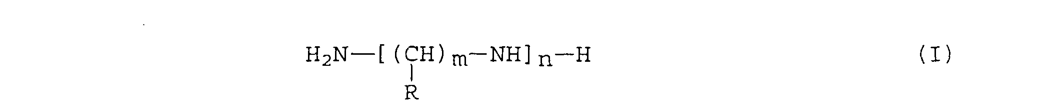 in der
- m eine ganze Zahl zwischen 2 und einschließlich 6 ist,
- n eine ganze Zahl zwischen 1 und einschließlich 9 ist, mit der Notwendigkeit einer einzigen Gruppe R, die von Wasserstoff verschieden und in der allgemeinen Formel anwesend ist, und variablen oder identischen Werten von m, das in den Gruppen ―(CH₂)ₘ― oder 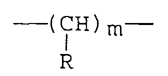 vorliegt,
- R ein Wasserstoffatom oder einen Rest der allgemeinen Formel (II) darstellt: 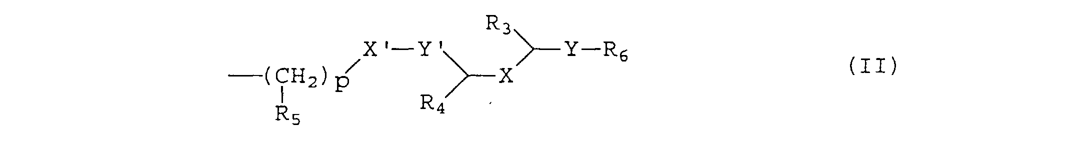 in der
- X und X' unabhängig voneinander ein Sauerstoffatom, eine Gruppe Methylen -(CH₂)_{q}- mit q gleich 0, 1, 2 oder 3 bedeuten oder eine Gruppe Amino -NH- oder -NR'- darstellen, worin R' eine Gruppe Alkyl mit 1 bis 4 Kohlenstoffatomen ist,
- Y und Y' unabhängig voneinander eine Gruppe Methylen, eine Gruppe Carbonyl oder eine Gruppe C=S bedeuten,
- R₃, R₄ und R₅ unabhängig voneinander ein Wasserstoffatom oder einen substituierten oder nicht substituierten Rest Alkyl mit 1 bis 4 Kohlenstoffatomen darstellen, wobei p zwischen 0 und 5 variieren kann,
- R₆ eine Gruppe Cholesterol oder eine Gruppe Alkylamino -NR₁R₂ bedeutet, worin R₁ und R₂ unabhängig voneinander einen gesättigten oder ungesättigten, geraden oder verzweigten Rest Alkyl mit 12 bis 22 Kohlenstoffatomen darstellen.

2. Lipopolyamin nach Anspruch 1, dadurch gekennzeichnet, daß n noch bevorzugter zwischen 1 und 5 ausgewählt wird.

3. Lipopolyamin nach Anspruch 1, dadurch gekennzeichnet, daß darin R vorzugsweise durch die allgemeine Formel (II') 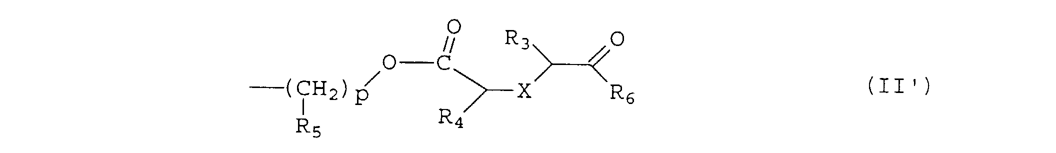 dargestellt wird, worin R₃, R₄, R₅, R₆ und p den in Anspruch 1 gegebenen Definitionen entsprechen und X ein Sauerstoffatom oder eine Gruppe - (CH₂)_{q}- mit q gleich null ist.

4. Lipopolyamin nach Anspruch 1 oder 3, dadurch gekennzeichnet, daß es sich um 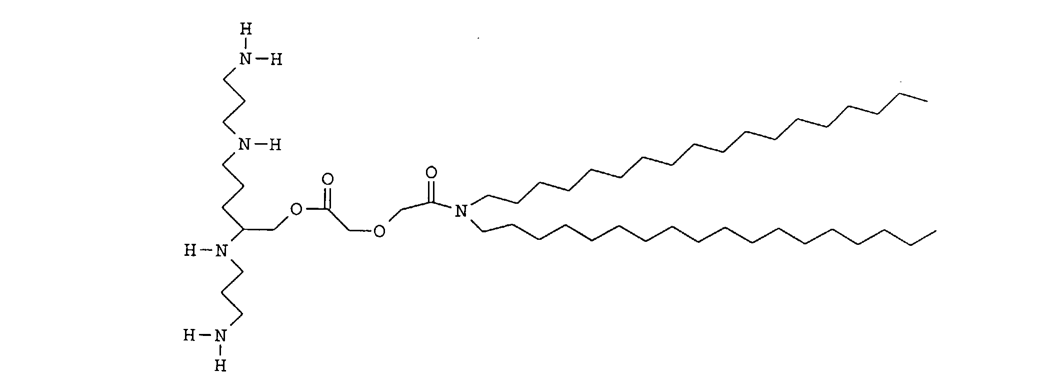 handelt, in der Form D, L, DL oder eines seiner Salze.

5. Lipopolyamin nach Anspruch 1 oder 3, dadurch gekennzeichnet, daß es sich um 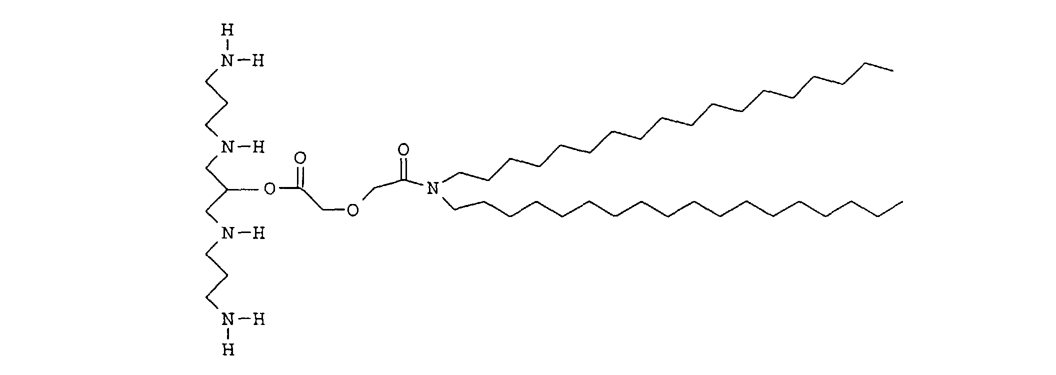 handelt, in der Form D, L, DL oder eines seiner Salze.

6. Lipopolyamin nach Anspruch 1 oder 3, dadurch gekennzeichnet, daß es sich um 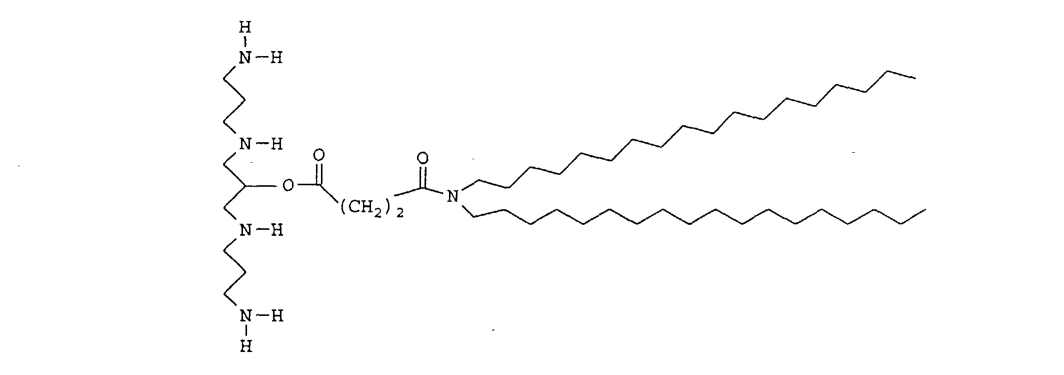 handelt, in der Form D, L, DL oder eines seiner Salze.

7. Lipopolyamin nach Anspruch 1 oder 3, dadurch gekennzeichnet, daß es sich um 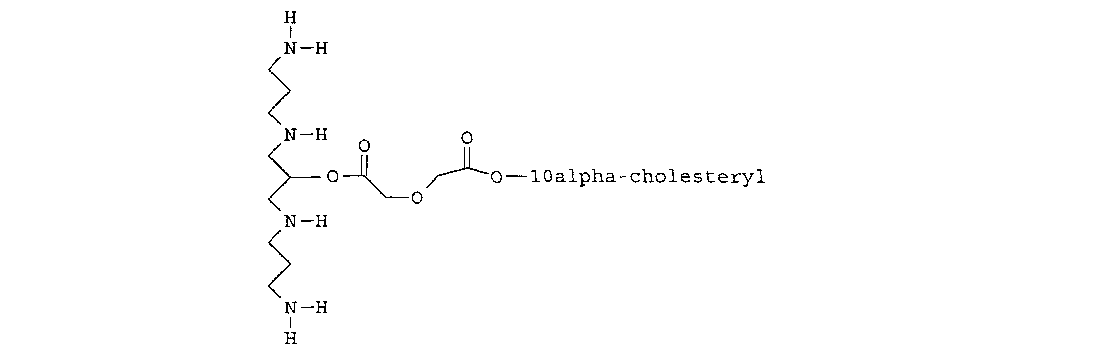 handelt, in der Form D, L, DL oder eines seiner Salze.

8. Pharmazeutische Zusammensetzung, dadurch gekennzeichnet, daß sie mindestens ein Lipopolyamin nach einem der Ansprüche 1 bis 7 und mindestens eine Nucleinsäure enthält.

9. Zusammensetzung nach Anspruch 8, dadurch gekennzeichnet, daß die Nucleinsäure eine Desoxyribonucleinsäure ist.

10. Zusammensetzung nach Anspruch 8, dadurch gekennzeichnet, daß die Nucleinsäure eine Ribonucleinsäure ist.

11. Zusammensetzung nach Anspruch 8, 9 oder 10, dadurch gekennzeichnet, daß die Nucleinsäure chemisch modifiziert ist.

12. Zusammensetzung nach einem der Ansprüche 8 bis 11, dadurch gekennzeichnet, daß die Nucleinsäure einen Gegensinn aufweist.

13. Zusammensetzung nach einem der Ansprüche 8 bis 11, dadurch gekennzeichnet, daß die Nucleinsäure ein therapeutisches Gen umfaßt.

14. Zusammensetzung nach Anspruch 13, dadurch gekennzeichnet, daß das therapeutische Gen für ein Protein kodiert, das in den Metabolismus der Lipide einbezogen ist, wie durch ein Apolipoprotein, ausgewählt unter den Apolipoproteinen A-I, A-II, A-IV, B, C-I, C-II, C-III, D, E, F, G, H, J und apo(a), ein Enzym wie Lipoprotein-Lipase, hepatische Lipase oder andere Lipasen, Lecithin-Cholesterol-Acyltransferase, 7-alpha-Cholesterol-Hydroxylase, phosphatidische saure Phosphatase, ein Transferprotein von Lipiden wie das Transferprotein der Cholesterol-Ester und das Transferprotein der Phospholipide, ein Bindungsprotein von HDL oder auch einen Rezeptor, beispielsweise ausgewählt unter den Rezeptoren LDL, den Rezeptoren von Chylomicron-Remnanten und den Rezeptoren Scavenger.

15. Pharmazeutische Zusammensetzung, umfassend eine Nucleinsäure, ein Lipopolyamin nach einem der Ansprüche 1 bis 7 und einen Zusatzstoff, der fähig ist, sich an den Komplex Lipopolyamin/ Nucleinsäure zu assoziieren und seine Fähigkeit zur Transfektion zu verbessern.

16. Zusammensetzung nach Anspruch 15, dadurch gekennzeichnet, daß der Zusatzstoff ein oder mehrere neutrale Lipide darstellt.

17. Zusammensetzung nach Anspruch 16, dadurch gekennzeichnet, daß das oder die neutralen Lipide unter den natürlichen oder synthetischen, zwitterionischen oder solchen Lipiden ausgewählt werden, die von ionischer Ladung unter physiologischen Bedingungen befreit wurden.

18. Zusammensetzung nach Anspruch 17, dadurch gekennzeichnet, daß das oder die neutralen Lipide solche mit zwei Fettketten sind.

19. Zusammensetzung nach Anspruch 18, dadurch gekennzeichnet, daß das oder die neutralen Lipide unter Dioleoylphosphatidyl-ethanol-amin (DOPE), Oleoylpalmitoyl-phosphatidyl-ethanolamin (POPE), Distearoyl, -palmitoyl, -miristoyl phosphatidyl-ethanolamin sowie ihren ein- bis dreimal N-methylierten Derivaten; den Phosphatidyl-glycerinen, den Diacyl-glycerinen, den Glycosyldiacyl-glycerinen, den Cerebrosiden (wie insbesondere den Galactocerebrosiden), den Sphingolipiden (wie insbesondere den Sphingomyelinen) und den Asialogangliosiden (wie insbesondere den asialoGM1 und GM2) ausgewählt werden.

20. Zusammensetzung nach einem der Ansprüche 15 bis 19, dadurch gekennzeichnet, daß sie 0,1 bis 20 Äquivalente Zusatzstoff pro 1 Äquivalent Lipopolyamin, und noch bevorzugter 1 bis 5 umfaßt.

21. Zusammensetzung nach irgendeinem der Ansprüche 8 bis 20, dadurch gekennzeichnet, daß sie einen pharmazeutisch akzeptablen Trägerstoff für eine injizierbare Formulierung umfaßt.

22. Zusammensetzung nach irgendeinem der Ansprüche 8 bis 20, dadurch gekennzeichnet, daß sie einen pharmazeutisch akzeptablen Trägerstoff für eine Anwendung auf der Haut und/oder den Schleimhäuten umfaßt.

23. Verwendung eines Lipopolyamins nach einem der Ansprüche 1 bis 7 zur Herstellung einer Zusammensetzung für die Transfektion von Zellen in vivo oder in vitro.

## Claims

1. Lipopolyamine in D, L or DL form or one of the salts thereof, characterized in that it is represented by the general formula I 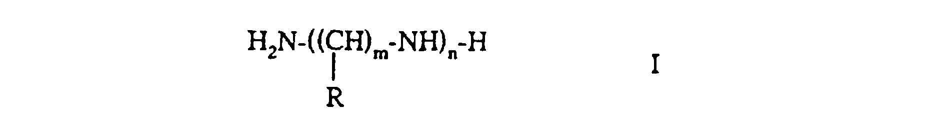 in which
- m is an integer between 2 and 6 inclusively,
- n is an integer between 1 and 9 inclusively with necessarily a single group R other than hydrogen present in the general formula and values of m which are variable or identical in the various groups -(CH₂)ₘ- or 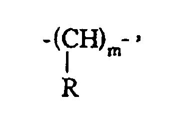
- R represents a hydrogen atom or a radical of general formula II 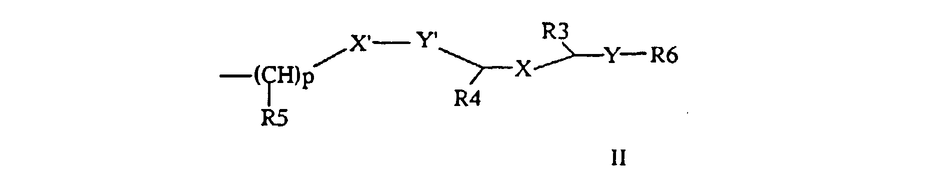 in which
- X and X' represent, independently of each other, an oxygen atom, a methylene group - (CH₂)_{q}- with q equal to 0, 1, 2 or 3, or an amino group -NH- or -NR'- with R' representing a C₁ to C₄ alkyl group,
- Y and Y' represent, independently of each other, a methylene group, a carbonyl group or a C=S group,
- R₃, R₄ and R₅ represent, independently of each other, a hydrogen atom or a substituted or unsubstituted C₁-C₄ alkyl radical, with it being possible for p to range between 0 and 5,
- R₆ represents a cholesterol group or an alkylamino group -NR₁R₂ with R₁ and R₂ representing, independently of each other, a linear or branched, saturated or unsaturated C₁₂ to C₂₂ aliphatic radical.

2. Lipopolyamine according to claim 1, characterized in that n is chosen more preferably from between 1 and 5.

3. Lipopolyamine according to claim 1, characterized in that R in it is preferably represented by the general formula II' 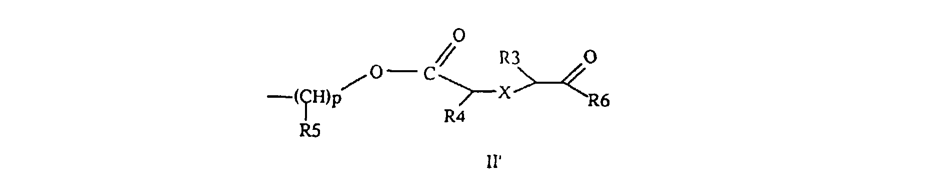 in which R₃, R₄, R₅, R₆ and p correspond to the definitions given in claim 1 and X represents an oxygen atom or a group -(CH₂)_{q}- with q being equal to zero.

4. Lipopolyamine according to claim 1 or 3, characterized in that it is 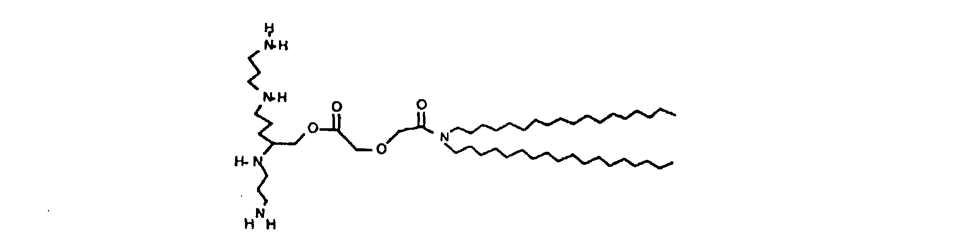 in D, L or DL form or one of the salts thereof.

5. Lipopolyamine according to claim 1 or 3, characterized in that it is 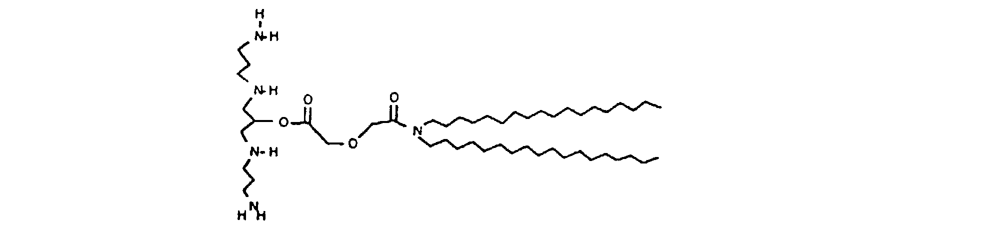 in D, L or DL form or one of the salts thereof.

6. Lipopolyamine according to claim 1 or 3, characterized in that it is 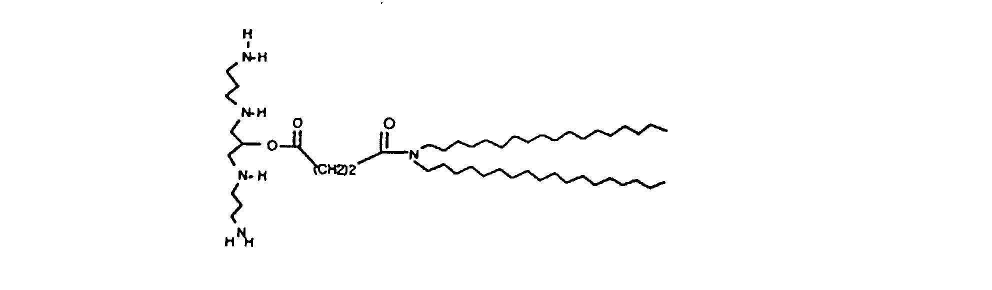 in D, L or DL form or one of the salts thereof.

7. Lipopolyamine according to claim 1 or 3, characterized in that it is 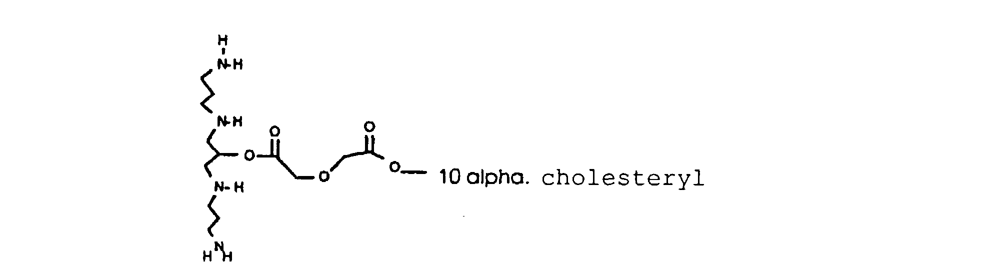 in D, L or DL form or one of the salts thereof.

8. Pharmaceutical composition, characterized in that it contains at least one lipopolyamine according to one of claims 1 to 7 and at least one nucleic acid.

9. Composition according to claim 8, characterized in that the nucleic acid is a deoxyribonucleic acid.

10. Composition according to claim 8, characterized in that the nucleic acid is a ribonucleic acid.

11. Composition according to claim 8, 9 or 10, characterized in that the nucleic acid is chemically modified.

12. Composition according to one of claims 8 to 11, characterized in that the nucleic acid is an antisense nucleic acid.

13. Composition according to one of claims 8 to 11, characterized in that the nucleic acid contains a therapeutic gene.

14. Composition according to claim 13, characterized in that the therapeutic gene encodes a protein involved in the metabolism of lipids such as an apolipoprotein chosen from the apolipoproteins A-I, A-II, A-IV, B, C-I, C-II, C-III, D, E, F, G, H, J and apo(a), an enzyme such as lipoprotein lipase, hepatic lipase or other lipases, lecithin cholesterol acyltransferase, 7-alpha-cholesterol hydroxylase, phosphatidic acid phosphatase, a lipid transfer protein such as the cholesterol ester transfer protein and the phospholipid transfer protein, an HDL-binding protein or a receptor chosen, for example, from LDL receptors, chylomicron remnant receptors and scavenger receptors.

15. Pharmaceutical composition comprising a nucleic acid, a lipopolyamine according to one of claims 1 to 7 and an adjuvant capable of associating with the lipopolyamine/nucleic acid complex and of improving its transfecting power.

16. Composition according to claim 15, characterized in that the adjuvant is one or more neutral lipids.

17. Composition according to claim 16, characterized in that the neutral lipid or lipids are chosen from synthetic or natural lipids, which may be zwitterionic or devoid of ionic charge under physiological conditions.

18. Composition according to claim 17, characterized in that the neutral lipid or lipids are lipids containing 2 fatty chains.

19. Composition according to claim 18, characterized in that the neutral lipid or lipids are chosen from dioleoylphosphatidylethanolamine (DOPE), oleoylpalmitoylphosphatidylethanolamine (POPE), di-stearoyl, -palmitoyl, -myristoyl phosphatidyl-ethanolamines as well as derivatives thereof N-methylated 1 to 3 times, phosphatidylglycerols, diacylglycerols, glycosyldiacylglycerols, cerebrosides (such as galactocerebrosides in particular), sphingolipids (such as sphingomyelins in particular) or alternatively asialogangliosides (such as asialoGM1 and GM2).

20. Composition according to one of claims 15 to 19, characterized in that it comprises from 0.1 to 20 equivalents of adjuvant per 1 equivalent of lipopolyamine and, more preferably, from 1 to 5.

21. Composition according to any one of claims 8 to 20, characterized in that it comprises a vehicle which is pharmaceutically acceptable for an injectable formulation.

22. Composition according to any one of claims 8 to 20, characterized in that it comprises a vehicle which is pharmaceutically acceptable for an application to the skin and/or the mucous membranes.

23. Use of a lipopolyamine according to one of claims 1 to 7 for preparing a composition for the in vivo or in vitro transfection of cells.
